# EUROPEAN PATENT APPLICATION

(11) **EP 1 847 224 A1**
(43) Date of publication of application: **24.10.2007**
(21) Application number: 06010027.8
(22) Date of filing: 20.04.2006
(51) Int. Cl.: A61B 17/04

(54) **Twisted cable for use in medical applications**

(71) Applicant: DSM IP Assets B.V., 6411 TE Heerlen (NL)
(72) Inventor: Oosterom, Rogier, 6221 JB Sittard (NL); Dirks, Christiaan Henri Peter, 3650 Dilsen (BE)
(74) Representative: Dorrestijn, Antoon

(57) **Abstract**

Cable for use in medical applications comprising ultra high molecular weight polyolefin filaments, which cable consists of between 3 and 8 strands, which strands are twisted to form the cable, the strands being twisted in a first direction, the largest structural elements in a strand being twisted to form the strand in a direction opposite to the first direction, the cable having a titer of between 9000 and 21000 dtex, the strands having a titer between 1000 and 6500 dtex.

## Description

The invention relates to a cable for use in medical applications comprising ultra high molecular weight polyolefin filaments. Good examples for such a cable include a trauma fixation cable, a sternum closure cable, a prophylactic or periprosthetic cable, long bone fracture fixation cable, small bone fracture fixation cable

WO-9617544 discloses a flexible cable for use in medical applications, as for instance use in bone fusion, bone fracture fixation and ligament reattachment. The cable is a braided cable, having a specific complicated structure.

WO-9415550 discloses a polymeric cable for repair of small bone fractures, ligaments and tendons. The cable has a hollow braided structure of ultra-high molecular weight polyethylene fibres. The cable has a complicated structure.

A disadvantage of the known cables is that their structure is complicated, so that it is difficult to produce the cables. Furthermore there is need for cables for use in medical applications having improved properties.

Aim of the invention is to provide such a cable that is simple to produce and yet shows good properties.

Surprisingly this object is obtained if the cable consists of between 3 and 8 strands, the strands being twisted in a first direction to form the cable, the largest structural elements in a strand being twisted in a direction opposite to the first direction to form the strand, the cable having a titer of between 9000 and 21000 dtex, the strands having a titer between 1000 and 6500 dtex.

It is very surprising that with a cable of such a rather simple structure very good results are obtained, whereas the known cables comprising the ultra high molecular weight polyolefin filaments since a long time all have a complicated structure, such as a braided structure rather than a twisted structure and often have a separate core and sheath structure, the sheath being braided around the core.

The cable according to the invention is very soft, well pliable and yet stronger than the known cables having the braided structure. Especially the tensile strength of the cable is very high, which is important in the heavy-duty medical applications the cables are used for.

Furthermore the cable has a smooth surface and a round intersection, so that it slides very easy through tissue when repairing a wound.

Moreover it shows high knot strength.

The cable according to the invention may comprise further components, for example a clamping device or a tensioning device.

High molecular weight polyolefin filaments are known to the skilled person. The filaments have an elongate body whose length dimension is greater than the transverse dimensions of width and thickness. It is preferred for the filaments to have a tensile strength of at least 1.2 GPa and a tensile modulus of at least 40 GPa.

Homopolymers and copolymers of polyethylene and polypropylene are particularly suitable as polyolefins for the production of the high molecular weight polyolefin fibres. Furthermore, the polyolefins used may contain small amounts of one or more other polymers, in particular other alkene-1-polymers.

Preferably the filaments are ultra high molecular weight polyethylene (UHMWPE) filaments, prepared by a gel spinning process, as for example described in EP 0205960 A, EP 0213208 A1, US 4413110, GB 2042414 A, EP 0200547 B1, EP 0472114 B1, WO 01/73173 A1, and Advanced Fiber Spinning Technology, Ed. T. Nakajima, Woodhead Publ. Ltd (1994), ISBN 1-855-73182-7, and references cited therein. Gel spinning is understood to include at least the steps of spinning at least one filament from a solution of ultra-high molecular weight polyethylene in a spin solvent; cooling the filament obtained to form a gel filament; removing at least partly the spin solvent from the gel filament; and drawing the filament in at least one drawing step before, during or after removing spin solvent. Suitable spin solvents include for example paraffins, mineral oil, kerosene or decalin. Spin solvent can be removed by evaporation, by extraction, or by a combination of evaporation and extraction routes. Such filaments are commercially available as Spectra^{®} or Dyneema^{®} grades.

Good results are obtained if the UHMWPE has an intrinsic viscosity (IV, as determined according to method PTC-179 (Hercules Inc. Rev. Apr. 29, 1982) at 135°C in decalin, with dissolution time of 16 hours, with anti-oxidant DBPC in an amount of 2 g/l solution, and the viscosity at different concentrations extrapolated to zero concentration) of above 5 dl/g. Particularly suitable is UHMWPE with IV of between about 8 and 40 dl/g, more preferably between 10 and 30, or 12 and 28, or between 15 and 25 dl/g. These ranges represent an optimum in polymer processability and filaments properties. Intrinsic viscosity is a measure for molar mass (also called molecular weight) that can more easily be determined than actual molar mass parameters like Mₙ and M_{w}. There are several empirical relations between IV and M_{w}, but such relation is highly dependent on molar mass distribution. Based on the equation M_{w} = 5.37 x 10⁴ [IV]^{1.37} (see EP 0504954 A1) an IV of 8 dl/g would be equivalent to Mw of about 930 kg/mol.

Preferably, the UHMWPE is a linear polyethylene with less than one branch or side chain per 100 carbon atoms, and preferably less than one side chain per 300 carbon atoms, a branch usually containing at least 10 carbon atoms. The linear polyethylene may further contain up to 5 mol% of one or more comonomers, such as alkenes like propylene, butene, pentene, 4-methylpentene or octene.

In a preferred embodiment, the UHMWPE contains a small amount of relatively small groups as side chains, preferably a C1-C4 alkyl group. It is found that a filament from UHMWPE with a certain amount of such groups show reduced creep behaviour. Too large a side chain, or too high an amount of side chains, however, negatively affects the processing and especially the drawing behaviour of the filaments. For this reason, the UHMWPE preferably contains methyl or ethyl side chains, more preferably methyl side chains. The UHMWPE therefore contains preferably at least 0.2, 0.3, 0.4 or 0.5 methyl or ethyl side chains. The amount of side chains is preferably at most 20, more preferably at most 10 per 1000 carbon atoms.

The UHMWPE can be a single polymer grade, but also a mixture of two or more different grades, e.g. differing in IV or molar mass distribution, and/or number of side chains.

The UHMWPE filaments may further contain usual amounts, generally less than 5 mass% of customary additives, such as anti-oxidants, thermal stabilizers, colorants, nucleating agents, flow promoters, catalyst residues etc.; as long as these components are suitable for the use in a surgical product. The UHMWPE filaments preferably contain less than 800 ppm of residual amounts of spin solvent, more preferably less than 500, 250, or even less than 100 ppm. The filaments may also contain other polymers, preferably polyolefinic polymers, like other polyethylenes, polypropylenes, or their copolymers, including rubbery copolymers like EPDM, EPR, etc. The amount of such other polymer is always lower than the amount of UHMWPE in the filament, and is preferably not more than 30 mass%, or more preferably not more than 20, 10 or 5 mass% of the UHMWPE filament.

A strand is the largest structural element in the cable according to the invention. A strand may consist of a monofilament, a yarn or two or more assembled yarns, forming as such the largest structural element in the strand. It is even possible that a strand consists of two or more sub-strands as the largest structural element in the strand, the sub-strands as such having the same structure as defined above for a strand. The strands are twisted together in a first direction to form the cable, the largest structural elements in a strand being twisted in a direction opposite to the first direction to form the strand. Therefore, in case a strand is formed by twisting together two or more yarns, the yarns are twisted together in an opposite to the twist direction in which the strands are twisted together to form the cable.

In case a strand consists of sub-strands, the sub-strands arte twisted together to form the strand in a direction opposite to the twist direction in which the strands are twisted to form the cable. It is possible that the strands in the cable are twisted in the Z-direction. In that case the largest structural elements in a strand will be twisted in the S-direction. It is also possible that the cable is twisted in the S-direction. In that case the largest structural elements in a strand will be twisted in the Z-direction. The opposite twist direction suppresses the unravelling of the cable.

The cable consists of between 3 and 8 strands.

In a preferred embodiment the cable consists of 3 or 4 strands, the strands having been twisted together to form the cable. In that case the cable slips very easy through a cable guide when the cable is applied for example when repairing a long bone fracture. The cable also has a very high strength.

The cable has a titer of between 9000 and 21000 dtex, the strands have a titer between 1000 and 6500 dtex. Therefore the cable is very suitable to be used in case of serious trauma or for bone fixation purposes.

Preferably the cable has a titer between 10000 and 19000 dtex, more preferably between 11000 mm and 13500 dtex. This provides a very good compromise between cable strength and patient comfort.

Preferably the strand has a titer between 1100 and 5500 dtex, more preferably between 1400 and 4600 dtex.

The cable may in addition to the ultra high molecular polyolefin filaments comprise further components, for example compounds that provide some functional effect, like anti-microbial or anti-inflammatory action, or that further improve knotting performance. The amount of such other components is generally limited to at most 20 mass% (relative to total cable mass), preferably at most 10, more preferably at most 5 mass%.

The cable according to the invention comprises preferably at least 50 mass% of the ultra high molecular weight polyolefin filaments. The ultra high molecular weight polyolefin filaments contribute most to the strength properties of the member. Furthermore the filaments enhance the sliding properties of the member through tissue. Therefore more preferably the cable comprises at least 60 mass% of ultra high molecular weight polyolefin filaments, more preferably at least 70, 80 or at least 90 mass%. The cable may further comprise other fibres, e.g. other biocompatible materials like polymers, to provide some other additional properties to the member, including improved knot slip behaviour or visual contrast. Such other fibres may be present in the form of one or more strands in the member. However, preferably each strand has the same composition, so that each strand comprises the same amount of the polyolefin filaments and of the other filaments. This ensures that the cable has a homogeneous structure, so that it is very well suitable to be used as a fixation cable.

Suitable examples of other fibrous materials include filaments or staple fibres made from non-absorbable polymers like other polyolefins, fluoropolymers, or semi-aromatic polyesters like polyethylene terephthalate, absorbable polymers like aliphatic polyesters based on e.g. lactides, or particles for X-ray visibility

Most preferably the cable consists of the polyolefin filaments.

Preferably the cable is treated at one or both of its ends to prevent unravelling. Good results are obtained if the treatment is carried out over a length of between 10 and 40 mm at an end of the cable. One very good way of treatment is a heat treatment, preferably while applying a tension to the cable, so that the filaments at the place of treatment are at least partly molten together.

The conditions for the treatment, like temperature, residence time and tension level are selected to be such that the filaments will soften or even start to melt, but without loosing their crystalline orientation. This allows them to combine and adhere to each other at least at their surface. The temperature at which the process is carried out is preferably within the range from about 150° C up to about 157° C for gel spun ultra high molecular weight polyethylene filaments. Residence times during which the precursor is exposed to these temperatures may be within the range from about 6 seconds to about 150 seconds.
The invention is further explained in the figures.
In Fig.1 an example of a cable according to the invention.

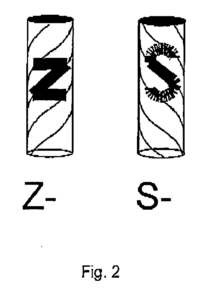

In Fig.2 the S and the Z twist directions are represented.

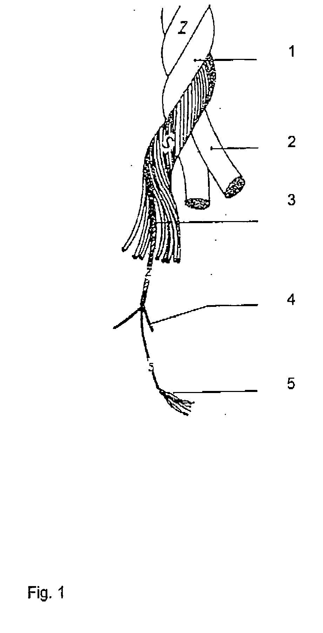

Fig. 1 shows a twisted cable according to the invention. The cable (1) consists of three strands (2). The strands are twisted together in the Z-direction to form the cable. A strand consists of a plurality of sub-strands (3), being twisted together in the S-direction to form the strand. Each sub-strand consists of three yarns (4) and (5).

The yarns are twisted together in the Z-direction to form the sub-strands, while each yarn as such is twisted in the S-direction.

### Comparative Experiment A

8 strands of a high molecular weight polethylene multifilament yarn of 1760 dtex, sold by DSM Dyneema in the Netherlands, having a tenacity of 35 cN/dtex and tensile modulus of 1368 cN/dtex, were braided on a Herzog braiding machine into a cable with 2 picks per centimetre.

Properties of the cable were determined applying following methods:
- Tensile properties: tensile strength (further indicated by tenacity), tensile modulus (further indicated by modulus) and elongation at break (further indicated by eab) are defined and determined with the procedure in accordance with ASTM D885M, using a nominal gauge length of the fibre of 3500 mm, a crosshead speed of 150 mm/min, and Zwick clamps for multifilament yarn. On the basis of the measured stress-strain curve the modulus is determined as the gradient between 0.3 and 1% strain. For calculation of the modulus and tenacity, the tensile forces measured are divided by the titer, as determined by weighing 10 metres of the member. Results are indicated in Table 1.

### Example 1

A cable according to the invention was made by twisting together 3 strands consisting of high molecular weight polyethylene filaments. The strands were assembled by twisting together 3 yarns, each with a titer of 880 dtex, with 139 turns per meter in the S-direction on a Saur Alma TT47 & Saur Alma TT06 twisting machine. Whereas the strands itself had been twisted together to form the cable with 80 turns per meter in the Z-direction. Properties were determined as indicated above; results are collected in Table 1.

**Table 1**

| Sample | Titer (dtex) | | Theoretical strength cable (N, 100% eff.) | Analysed strength cable (N) | Efficiency linear strength (%) |
|---|---|---|---|---|---|
| | Strand | cable | | | |
| Comp. Exp. 1 | 1760 | 14450 | 5058 | 3482 | 69 |
| Ex. 1 | 2640 | 8083 | 2467 | 2100 | 85 |

As can be concluded from comparing comparative experiment A and example, 1 the cable according to the invention has lost 15% whereas the conventional cable looses up to 31% of its theoretical strength.

## Claims

1. Cable for use in medical applications comprising ultra high molecular weight polyolefin filaments, **characterized in that** cable consists of between 3 and 8 strands, the strands being twisted in a first direction to form the cable, the largest structural elements in a strand being twisted in a direction opposite to the first direction to form the strand, the cable having a titer of between 9000 and 21000 dtex, the strands having a titer between 1000 and 6500 dtex.

2. Cable according to claim 1, **characterized in that** the ultra high molecular weight polyolefin filaments are ultra high molecular weight polyethylene filaments made by a gel spinning process.

3. Cable according to any one of claims 1 or 2, **characterized in that** the cable comprises at least 50 mass% of the ultra high molecular weight polyolefin filaments.

4. Cable according to any one of claims 1 - 3, **characterized in that** the cable consists of between 3 or 4 strands.

5. Cable according to any one of claims 1-4, **characterized in that** the titer of the cable is between 10000 and 19000 dtex.

6. Cable according to any one of claims 1 - 5, **characterized in that** the titer of a strand is between 1100 and 5500 dtex.

7. Cable according to any one of claims 1 - 6, **characterized in that** the cable comprises at least 50 mass % of the polyolefin filaments.

8. Cable according to any one of claims 1 - 7, **characterized in that** the member is treated at one or both of its ends to prevent unraveling.

9. Cable according to claim 8, **characterized in that** as treatment a heat treatment is applied.
